Europäisches Patentamt

European Patent Office

Office européen des brevets

(11) Numéro de publication: **0 041 426**
**B1**

(12) **FASCICULE DE BREVET EUROPEEN**

(45) Date de publication du fascicule du brevet: **13.11.85**

(21) Numéro de dépôt: **81400777.9**

(22) Date de dépôt: **18.05.81**

(51) Int. Cl.⁴: **G 01 N 33/53,** G 01 N 33/58

(54) Produit de couplage entre une lectine et un ligand spécifique, obtention et application dans le domaine biologique.

(30) Priorité: **22.05.80 FR 8011470**
**15.07.80 CH 5428/80**

(43) Date de publication de la demande:
**09.12.81 Bulletin 81/49**

(45) Mention de la délivrance du brevet:
**13.11.85 Bulletin 85/46**

(84) Etats contractants désignés:
**BE DE FR GB IT NL**

(56) Documents cités:
**EP-A-0 033 437**
**WO-A-80/02296**
**DE-A-2 732 554**
**DE-A-2 951 678**
**DE-A-2 952 373**
**FR-A-1 516 640**
**FR-A-2 334 107**
**FR-A-2 418 463**

IMMUNOCHEMISTRY, vol. 6, 1969, Pergamon Press (GB) S. AVRAMEAS: "Coupling of enzymes to Proteins with Glutaraldehyde", pages 43-52

(73) Titulaire: **INSTITUT PASTEUR**
**25-28, rue du Docteur Roux**
**F-75724 Paris Cedex 15 (FR)**

(72) Inventeur: **Avrameas, Stratis**
**92 Elysées 2**
**F-78170 La Celle Saint Cloud (FR)**
Inventeur: **Guesdon, Jean-Luc**
**12, rue du Hameau**
**F-75015 Paris (FR)**

(74) Mandataire: **Phélip, Bruno et al**
**c/o Cabinet Harlé & Phélip 21, rue de la Rochefoucauld**
**F-75009 Paris (FR)**

(56) Documents cités:
**CHEMICAL ABSTRACTS, vol. 81, no. 11, 16 septembre 1974, abrégé 61897s, page 335, COLUMBUS, OHIO (US) S. LEMIEUX et al.: "Local hemolysis plaque assay using a new method of coupling antigens on sheep erythrocytes by glutaraldehyde"**

# 0 041 426

## Description

La présente invention concerne un produit de couplage entre une lectine et un ligand spécifique, son obtention et ses applications dans les techniques biologiques, notamment de localisation, de mise en évidence et de dosage d'anticorps ou d'antigènes selon les revendications 1, 6 et 9. Le produit de couplage selon l'invention est particulièrement approprié comme réactif universel dans les différents types de dosages biologiques et notamment immunologiques.

En immunologie, on a déjà utilisé, notamment, des produits de couplage d'anticorps ou d'antigènes avec différentes substances, à titre de marqueurs, telles que par exemple, les enzymes, les radioisotopes, la ferritine, les globules rouges, les fluorochromes. De tels produits de couplage sont utilisés à titre de réactifs selon divers procédés immunologiques pour la localisation, la mise en évidence ou le dosage des constituants humoraux ou cellulaires.

Pour plus de détails sur ces produits de couplage connus, on pourra se référer à l'ouvrage ci-après: "Handbook of experimental immunology", 3 vol. Ed. D. M. Weir (1978) Blackwell Scientific.

On a maintenant trouvé une nouvelle série de produits de couplage qui peuvent être utilisés dans un grand nombre de domaines; il s'agit des produits de couplage entre une lectine et un ligand spécifique.

La présente invention concerne donc, à titre de produit nouveau, un produit de couplage entre une lectine et un ligand spécifique choisi parmi les antigènes, les anticorps, les haptènes, les hormones et leurs récepteurs, les enzymes ou leurs inhibiteurs, les lectines.

Bien que les indications détaillées qui suivent concernent certaines lectines particulières, il est à noter que l'enseignement de la présente description est valable pour n'importe quelle lectine.

Les lectines sont des protéines extraites à partir de diverses sources, entre autres des plantes, qui réagissent d'une façon spécifique et par des liaisons non covalentes avec certains résidus glycosidiques.

A titre d'exemples de lectines on peut citer: la Concanavaline A extraite de *Canavalia-ensiformis*; la Ricine extraite de *Ricinus communis*, la lectine extraite de *Triticum vulgare*, c'est-à-dire l'agglutinine de germe de blé. On trouvera une description détaillée des lectines dans les ouvrages ci-après qui sont cités à titre de références dans la présente description:

— Science, 1972, vol. 177, p. 949—959,
— Annales d'Immunologie, 1979, vol. 1c No. 1, p. 4—16.


On indiquera que la Concanavaline A réagit de façon spécifique avec le méthyl-α-D-mannoside, la Ricine avec le D-galactose, tandis que la lectine extraite de *Triticum vulgare* réagit spécifiquement avec la N-acétylglucosamine.

Parmi les lectines, la Concanavaline A est celle qui possède la plus vaste spécificité et qui peut ête isolée facilement et en grande quantité. Elle est donc commode et peu coûteuse à utiliser. La lectine W. G. A. donne aussi d'excellents resultats. On peut utiliser, selon l'invention, une lectine quelconque; le choix de la lectine sera fonction de sa disponibilité et aussi de sa spécificité.

Par "ligand spécifique" on désigne dans la présente description toute substance soluble qui peut réagir de façon spécifique avec une autre substance biologique ou particulaire.

Par "substance soluble", on désigne toute substance soluble dans les milieux couramment utilisés pour les réactions biologiques. Il peut s'agir de milieux aqueux, tels que des milieux physiologiques, ou de mélanges de milieux aqueux et organiques.

De plus, le ligand spécifique mis en oeuvre selon l'invention doit être tel que le produit de couplage lectine-ligand selon l'invention soit soluble en milieu aqueux, en présence ou non du sucre spécifique de la lectine considérée, selon l'application dans laquelle ledit produit de couplage est mis en oeuvre.

Par "milieux aqueux" on désigne selon l'invention les milieux aqueux, tamponnés ou non, couramment utilisés dans le domaine biologique, tels que les solutions tampon phosphate; les solutions tampons contenant un détergent, tel que le "Tween", ou de la gélatine, la sérum albumine bovine, la lactalbumine bovine et autres substances habituellement mises en oeuvre dans de tels domaines.

Les ligands spécifiques répondant à une telle définition sont notamment les anticorps, les antigènes macromoléculaires ou les haptènes, les hormones et leurs récepteurs, les enzymes et leurs inhibiteurs, les lectines. Parmi les ligands spécifiques cités ci-dessus, on utilise le plus communément les anticorps et les antigènes.

Le produit selon l'invention est obtenu par couplage d'une lectine avec le ligand par l'intermédiaire d'un agent de couplage approprié, tel que par exemple le glutaraldéhyde et la benzoquinone. Le couplage est avantageusement réalisé par liaisons covalentes.

Le couplage lectine-ligand spécifique est réalisé selon un procédé analogue à ceux utilisés pour le couplage de protéines.

Par exemple, on peut réaliser le couplage lectine-ligand spécifique par un procédé similaire à celui décrit pour l'obtention de conjugués anticorps-enzyme dans le Scand. J. Immunol., vol. 8 suppl. 7 p. 7—23, 1978. Un tel procédé de couplage consiste à mettre en contact, en une ou plusieurs étapes, un agent de couplage avec les substances à coupler.

Lorsqu'on utilise la benzoquinone comme agent de couplage on peut indifféremment activer en

# 0 041 426

premier la lectine ou le ligand spécifique. A titre de document illustrant la technique de couplage à la benzoquinone, on peut également citer le brevet FR 75.37.392 (publication No. 2.334.107).

Lorsqu'on utilise le glutaraldéhyde comme agent de couplage, on mélange avantageusement la lectine et le ligand spécifique et on ajoute audit mélange le glutaraldéhyde en quantités appropriées pour réaliser le couplage. On peut cependant également réaliser ce couplage en deux temps, en mettant en contact avec le glutaraldéhyde l'un des réactifs, en éliminant le glutaraldéhyde en excès et en ajoutant l'autre réactif. On opère sensiblement selon le même mode opératoire que celui décrit dans l'article de S. Avrameas (Immunochemistry vol. 6 1969).

Dans de nombreux cas, le glutaraldéhyde convient comme agent de couplage, par exemple avec des antigènes ou des anticorps. Le tableau qui suit illustre la préparation de conjugués anticorps-lectine en utilisant le glutaraldéhyde comme agent de couplage. Cependant, ces exemples ne sont pas exhaustifs.

## TABLEAU 1

| Lectine | | Quantité d'anticorps ≠ | Volume de glutaraldéhyde (1%) |
|---|---|---|---|
| Origine | Quantité | | |
| *Lens culinaris* | 1,5 mg | 0,7 mg | 20 µl |
| *Triticum vulgare* | 0,7 mg | 0,4 mg | 20 µl |
| *Ricinus communis* | 1,7 mg | 0,9 mg | . 20 µl |
| *Canavalia ensiformis* | 4 mg | 2 mg | 20 µl |

≠ l'anticorps utilisé était de l'anticorps de mouton anti-immunoglobuline de lapin.

La lectine et l'anticorps sont dissous dans un tampon phosphate pH 6,8 0,1 M; à ce mélange on ajoute 20 µl de glutaraldéhyde à 1%. Le volume total du milieu de réaction est de 1 ml.

Mais, lorsque la lectine ne possède pas de groupements amines primaires, le glutaraldéhyde ne peut pas être mis en oeuvre pour le couplage. Dans ce cas, d'autres agents de couplage peuvent être utilisés. Ceux-ci doivent permettre la fixation de la lectine sur un autre ligand sans perte importante d'activité de l'un ou/et l'autre constituant.

Ainsi, avec la succinyl-concanavaline A, qui ne possède plus de groupements amines primaires, le glutaraldéhyde n'est pas utilisable mais la p-benzoquinone permet d'obtenir un conjugué anticorps-succinyl-concanavaline efficace.

Le produit de couplage lectine-ligand spécifique, selon l'invention, peut être caractérisé par sa capacité à réagir avec une substance spécifique et en même temps avec un marqueur spécifique de la lectine. Par exemple, si le produit de couplage lectine-ligand est un produit lectine-antigène, il pourra être caractérisé par sa capacité à réagir avec la substance spécifique correspondante, à savoir l'anticorps correspondant, et par sa capacité à réagir avec un marqueur spécifique de la lectine, par exemple avec une enzyme glycoprotéinique.

Il peut avantageusement être conservé en solution aqueuse à basse température, par exemple à 4°C.

On peut également le mélanger avec du glycerol (en général 50/50) et le conserver ainsi à 4°C ou au congélateur à −20°C.

Le produit de couplage lectine-ligand spécifique selon l'invention convient comme réactif dans divers procédés immunologiques de localisation, de mise en évidence et de dosage de substances biologiques, tels que les dosages immunologiques du type non compétitif, à double anticorps ou par compétition. Il est avantageusement mis en oeuvre sous la forme d'une solution aqueuse, tamponnée ou non, pouvant contenir un excès du sucre de faible poids moléculaire, spécifique de la lectine du produit de l'invention.

Il faut noter que les lectines ont déjà été proposées pour la détection des cancers par détermination de l'activité galactosyl-transférase dans les cellules. A cet effet on pourra se référer à la demande de brevet WO 80/02.296 qui concerne un procédé amélioré pour détecter les cancers par détermination de l'activité galactosyl-transférase dans les cellules à l'aide d'un marqueur spécifique de cette activité. Le marqueur mis en oeuvre doit avoir une activité spécifique pour la galactose ou les résidus galactose. Un exemple de marqueur approprié pour ce procédé est constitué par une lectine couplée avec un colorant, par exemple un colorant fluorescent. Dans ce procédé, c'est la lectine qui intervient dans la détermination de l'activité recherchée. Au contraire, dans les procédés immunologiques cités précédemment dans lesquels on peut mettre en oeuvre le produit de couplage lectine-ligand selon l'invention, c'est le ligand spécifique qui réagit avec la substance à doser et non pas la lectine comme c'est le cas dans le procédé selon la demande de brevet WO 80/02296.

Le produit de couplage lectine-ligand, selon l'invention, sous la forme d'une solution aqueuse contenant un excès d'un sucre de faible poids moléculaire, spécifique de la lectine, convient particulièrement bien à titre de réactif dans les techniques immunologiques, dans lesquelles l'un des réactifs ou la substance à identifier est insoluble ou immobilisée sur un support insoluble. Il convient donc en particulier dans tous les types de dosages mettant en oeuvre un support insoluble. Mais le produit de l'invention peut aussi être utilisé en phase soluble.

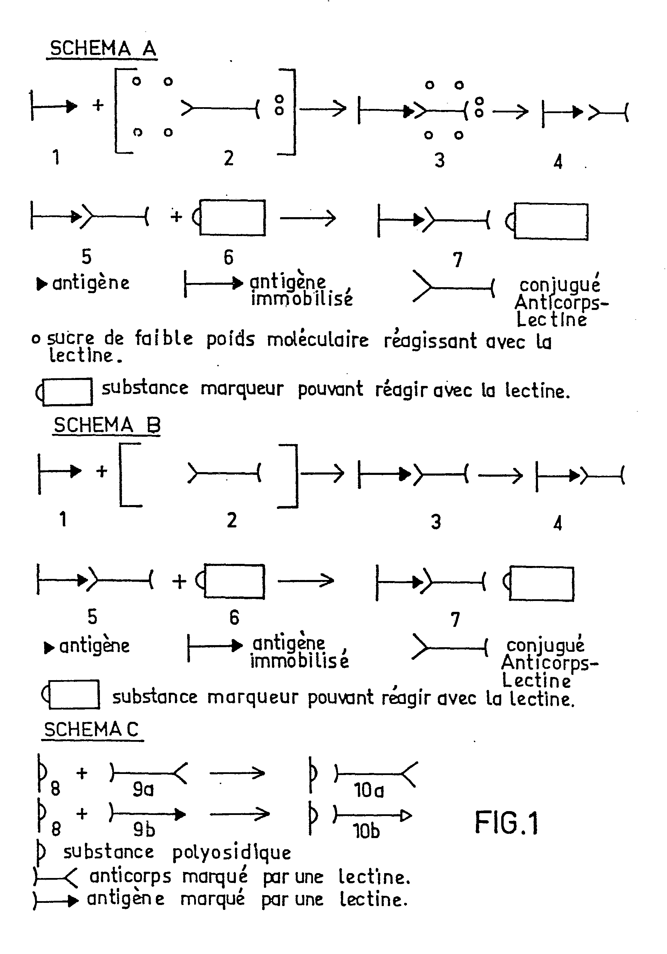

A titre d'exemple illustratif, on a indiqué sur la figure 1 annexée, le schéma d'un procédé de mise en évidence d'un antigène immobilisé (schéma A).

Pour mettre en évidence l'antigène immobilisé (1), on ajoute le produit de couplage lectine-ligand (2) selon l'invention, dissous dans un milieu aqueux contenant un sucre de faible poids moléculaire spécifique de la lectine du produit de couplage, le ligand étant dans ce cas particulier un anticorps.

La partie anticorps du produit de couplage (2) réagit avec l'antigène immobilisé, tandis que la partie lectine dudit produit de couplage, du fait de la présence du sucre en excès, ne peut réagir avec d'autres substances glycosidiques qui pourraient éventuellement être présentes dans le milieu réactionnel.

L'étape suivante de ce procédé consiste à laver le complexe antigène-produit de couplage (3) afin d'éliminer le sucre et l'excès de produit de couplage (2). On ajoute ensuite un marqueur portant une partie polyosidique pouvant réagir avec la lectine. Le marqueur est ensuite mis en évidence par tout moyen approprié, après lavage éventuel.

En variante, on peut également mettre en évidence l'antigène selon le schéma réactionnel B représenté sur la figure 1 annexée. Dans ce cas, le produit de couplage lectine-ligand est utilisé en solution en l'absence de sucre spécifique de la lectine. Il faut noter toutefois, que ce schéma réactionnel ne peut être utilisé que lorsque l'antigène à mettre en évidence n'est pas une substance portant des groupes glycosidiques susceptibles de réagir avec la lectine.

Dans le procédé de mise en évidence d'un antigène ci-dessus, la lectine liée à l'anticorps joue le rôle d'accepteur de marqueur. Etant donné qu'un grand nombre de marqueurs possède des parties polysaccharidiques ou que des résidus saccharidiques peuvent leur être facilement fixés, le même produit de couplage lectine-anticorps ou lectine-antigène peut être utilisé en combinaison avec un grand nombre de marqueurs. Par "marqueur", on désigne, selon l'invention, toute substance de nature quelconque, qui permet de faire une identification ou un dosage. A titre d'exemples de marqueurs, on citera les enzymes, les dérivés fluorescents, les éléments radioactifs, les globules rouges et produits similaires.

Certaines enzymes qui peuvent être utilisées comme marqueurs ne sont pas des glycoprotéines; cependant, il est possible, pour permettre leur utilisation avec un produit de couplage selon l'invention dans des procédés de dosage immunoenzymatiques, de coupler ou de fixer sur ces enzymes des fractions glycosidiques. Un moyen pour fixer de telles fractions consiste à greffer chimiquement une copule glucidique sur l'enzyme. Un autre moyen consiste à coupler une enzyme holoprotéique (par exemple la β-galactosidase) avec une glycoprotéine (par exemple la péroxydase ou la glucose oxydase). Par exemple, on peut préparer une glucose oxydase-β-galactosidase en mélangeant 2,5 mg (230 μl) de glucose oxydase, 4,1 mg (410 μl) de β-galactosidase et 50 μl de glutaraldéhyde à 1%. On peut donc utiliser n'importe quel marqueur dans le mesure où celui-ci possède des fractions glycosidiques ou peut être couplé ou fixé sur de telles fractions.

Le marqueur peut être également une substance portant un fluorochrome ou un isotope radioactif ou une substance particulaire pouvant réagir avec une lectine.

A chacun des marqueurs ci-dessus correspond une technique immunologique qui est respectivement la technique immunoenzymatique (dosage immunoenzymatique ou détection histochimique), l'immunofluorescence (dosage immunofluorimétrique ou détection histochimique) la technique radioimmunologique, l'hémagglutination et autres techniques de ce genre.

A titre d'exemple non limitatif, on décrira ci-après un procédé de dosage mettant en oeuvre un produit de couplage lectine-ligand selon l'invention.

Ce procédé de dosage pour la détermination d'une substance biologique donnée, consiste:

1) à immobiliser une substance ayant une affinité de fixation vis-à-vis de la substance biologique à doser.

2) à faire incuber cette substance avec le milieu contenant la substance biologique à doser;

3) à faire incuber, après lavage, le milieu réactionnel résultant avec un produit de couplage lectine-ligand spécifique, en solution dans un milieu aqueux contenant un excès d'un sucre spécifique de la lectine, ledit ligand étant capable de réagir de façon spécifique avec ladite substance ayant une affinité pour la substance biologique à doser ou avec ladite substance biologique elle-même;

4) à laver le milieu réactionnel résultant et à le faire incuber avec un marqueur portant des fractions glycosidiques pouvant réagir avec la lectine;

5) à révéler le marqueur par un moyen approprié.

Ce procédé est approprié pour doser des antigènes, des anticorps, des immunoglobulines et autres substances d'intérêt biologique.

La substance ayant une affinité de fixation vis-à-vis de la substance biologique à doser peut être toute substance capable de se fixer de façon spécifique avec ladite substance biologique. Par exemple, si la substance biologique à doser est un anticorps, la substance ayant une affinité de fixation sera alors un antigène et réciproquement.

On peut utiliser selon ce procédé un marqueur quelconque, tel que ceux cités ci-dessus, notamment

4

# 0 041 426

les enzymes et les globules rouges, auquel cas l'étape 5) est une étape de détermination enzymatique ou d'érythroadsorption.

Le produit de couplage lectine-ligand, selon l'invention, trouve une application particulièrement intéressante dans les techniques immunologiques mettant en oeuvre des globules rouges, comme marqueurs. En effet, dans les techniques connues d'hémagglutination, il est nécessaire d'utiliser des globules rouges sensibilisés par un antigène, par exemple. Le produit de couplage selon l'invention permet d'éviter cette sensibilisation puisque les globules rouges sont utilisés comme marqueurs pour révéler la présence du produit de couplage lectine-ligand spécifique et ceci par l'intermédiaire de la lectine. Le procédé de dosage selon l'invention impliquant l'utilisation de globules rouges peut être dénommé érythroadsorption spécifique. De plus, en utilisant ce procédé d'érythroadsorption, la quantité de globules rouges à mettre en oeuvre est beaucoup moins importante que dans les procédés d'hémagglutination classiques.

Les milieux contenant la substance biologique à doser sont généralement des sérums. Etant donné que les sérums présentent d'une espèce à l'autre des réactions croisées et qu'ils contiennent souvent des anticorps anti-globules rouges, par exemple des anticorps anti-globules rouges de mouton, il faudra prendre soin ''d'absorber'' avec des globules rouges le sérum à tester pour éviter les réactions faussement positives dans le cas où l'on utilise l'érythroadsorption. Bien qu'une telle technique fasse appel à des moyens connus de l'homme de l'art, on indiquera ci-après certaines de ses variantes de mise en oeuvre.

Dans le cas où l'anticorps ou l'antigène à doser n'est pas thermolabile, cette absorption pourra être effectuée en soumettant le sérum aux étapes ci-après qui consistent:

a) à décomplémenter le sérum, c'est-à-dire le chauffer à 56°C environ pendant environ 30 min.

b) à ajouter un excès de globules rouges de mouton audit sérum, par exemple pour obtenir une concentration de 10% de globules rouges;

c) à laisser le sérum à la température ambiante pendant environ 1 heure sous agitation douce;

d) à centrifuger pour récupérer le sérum purifié et éliminer le culot de globules rouges.

Dans le cas où l'antigène ou l'anticorps à doser est thermolabile, le sérum ne sera pas décomplémenté mais il sera absorbé par des globules rouges, par exemple de mouton, traités par un agent tannant, de préférence le glutaraldéhyde.

Le sérum ainsi traité peut ensuite être soumis au procédé de dosage selon l'invention, l'étape 5 consistant alors en une détermination d'érythroadsorption.

La détermination de l'érythroadsorption peut se faire de plusieurs façons. Par exemple, on peut constater visuellement que les globules rouges sont adsorbés à la surface du support sur lequel a été immobilisée la substance ayant une affinité de fixation pour la substance biologique à doser. Dans ce cas, le procédé de l'invention permet d'identifier dans un liquide biologique donné une substance biologique particulière. Si, au contraire, le liquide biologique ne contient pas la substance biologique particulière, les érythrocytes ne sont pas adsorbés et forment un culot au fond du récipient, par exemple des puits de microplaques. Le procédé de l'invention permet aussi de déterminer quantitativement une substance biologique donnée. A cet effet, on élimine les globules rouges n'ayant pas réagi, par exemple par aspiration à la pipette. Puis on lyse les érythrocytes adsorbés, par exemple à l'eau distillée, et on dose les substances libérées par les globules rouges, par exemple l'hémoglobine ou les substances artificiellement introduites par l'expérimentateur, par spectrophotométrie à 414 nm de préférence, ou à une longueur d'onde voisine, ce qui permet d'automatiser entièrement ce procédé de dosage.

L'hémoglobine peut également être dosée par une réaction enzymatique. Par exemple, on peut utiliser un des substrats de la peroxydase, tels que l'ortho-dianisidine ou l'ortho-phénylène-diamine. La lecture se fait aussi par spectrophotométrie à 400 nm pour l'ortho-dianisidine et à 492 nm pour l'ortho-phénylène-diamine.

La quantité de substances libérées par les globules rouges, par exemple la quantité d'hémoglobine libérée, est proportionnelle à la quantité de substance à doser, ce qui permet d'obtenir, par exemple, le dosage d'un antigène ou d'un anticorps présent dans l'échantillon en se référant à une gamme étalon d'hémolyse des globules rouges établie dans les mêmes conditions.

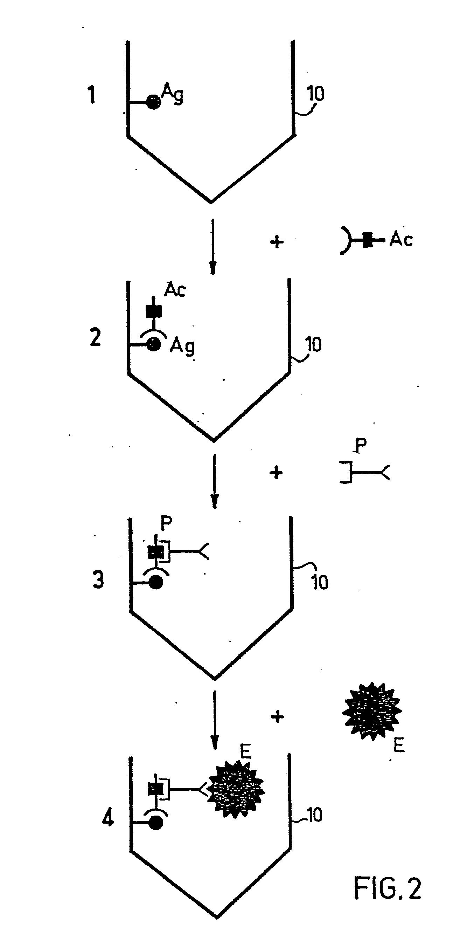

Le procédé de dosage par érythroadsorption selon l'invention est particulièrement approprié pour le dosage des anticorps et des antigènes et sera décrit plus en détail ci-après, sans en limiter pour autant la portée, en référence à la figure 2 annexée sur laquelle est représenté le schéma réactionnel de ce mode opératoire. Sur cette figure 2, (1) représente la première étape du procédé de l'invention au cours de laquelle on immobilise sur le support (10) des antigènes (Ag). Dans le cas particulier de la figure, le support (10) représente un puits en V d'une microplaque. L'immobilisation des antigènes, qui constituent dans ce cas particulier la substance ayant une affinité pour la substance biologique à doser, à savoir l'anticorps, est réalisée par exemple par adsorption passive ou, si nécessaire, par liaison covalente selon la nature du support.

L'étape (2) consiste en une incubation de l'antigène immobilisé avec le liquide biologique contenant l'anticorps (Ac) à doser, par exemple le sérum à titrer. Après cette étape d'incubation, au cours de laquelle l'antigène (Ag) interagit avec l'anticorps (Ac) correspondant, s'il est présent dans le sérum à tester, on lave le support à l'aide d'une solution tampon, par exemple une solution de tampon phosphate contenant éventuellement un détergent tel que le ''Tween'' dénommé ci-après PBS ou PBS-Tween.

L'étape (3) du procédé de l'invention consiste à incuber le support résultant de l'étape (2) avec un

5

produit de couplage ligand spécifique-ligand (P) capable de réagir avec des érythrocytes (E). Dans ce cas particulier, le ligand spécifique est un anticorps dirigé contre les immunoglobulines de l'espèce humaine ou animale du sérum à titrer. Après cette incubation, on lave le système résultant comme décrit ci-dessus pour éliminer le produit de couplage n'ayant pas réagi. Puis on ajoute des érythrocytes, qui sont adsorbés par le produit de couplage seulement si le sérum à titrer contient l'anticorps correspondant à l'antigène immobilisé, sinon les érythrocytes ne sont pas fixés et tombent au fond du récipient.

Le dosage quantitatif, c'est-à-dire la détermination de la quantité d'anticorps contenue dans le sérum, sera aisément effectué comme décrit ci-dessus, c'est-à-dire par lyse des érythrocytes et détermination de la quantité de substance libérée par ces érythrocytes, par exemple la quantité d'hémoglobine.

L'utilisation de lectines hémolysantes, pour l'obtention du produit de couplage selon l'invention, peut supprimer cette dernière étape de lyse des globules rouges.

Sur la figure 2, on a représenté le schéma réactionnel du procédé de dosage selon l'invention, utilisant des érythrocytes comme marqueurs (c'est-à-dire le procédé par érythroadsorption). Le procédé de l'invention utilisant un autre marqueur que des érythrocytes peut être représenté par le même schéma réactionnel si ce n'est qu'au lieu d'ajouter des érythrocytes on ajoute un autre marqueur.

A titre de support, on peut utiliser n'importe quel support insoluble présenté sous une forme définie, telle qu'une plaque ou une feuille, ou sous une forme particulière. A titre de substance constitutive de tels supports, on peut utiliser la cellulose et ses dérivés, le polyacrylamide, les polyméthacrylates d'alkyle et autres polymères d'origine naturelle ou synthétique ainsi que le verre. Toutefois, on notera que dans le cadre de la variante préférée selon l'invention, à savoir le procédé de dosage par érythroadsorption, on utilisera un support qui peut être mis sous la forme de récipient.

On utilise avantageusement des microplaques pour immobiliser l'antigène ou l'anticorps mis en oeuvre selon le procédé ci-dessus, par exemple des microplaques en polystyrène. On a constaté que les microplaques présentant des cavités à section en U ou en V étaient les mieux appropriées pour le procédé de dosage par érythroadsorption.

Pour la mise en oeuvre des autres variantes du procédé de l'invention, consistant à utiliser un marqueur autre que des érythrocytes, on peut également utiliser comme supports des gels, de préférence des gels magnétiques, tels que ceux décrits dans le brevet FR—A—2.334.106) et la demande de brevet FR 79.21.343.

On notera que le produit de couplage selon l'invention, utilisé sous la forme de solution, procure des avantages considérables dans le domaine des dosages immunologiques en ce sens que sa mise en oeuvre ne requiert aucune étape de séparation, centrifugation, filtration, autre que celle imposée par le support d'immobilisation de l'un des réactifs (par exemple, lavage du support ou séparation à l'aide d'un aimant dans le cas de gels magnétiques).

La sensibilité du procédé de dosage défini ci-dessus est identique à celle des procédés de dosages immunoenzymatiques classiques, c'est-à-dire de l'ordre de 1 ng pour les antigènes et de 10 à 20 ng pour les anticorps. Cette sensibilité est également du même ordre de grandeur qu'en radioimmunologie quantitative.

Le produit de couplage lectine-ligand spécifique selon l'invention convient également pour lier des antigènes (9b) ou des anticorps (9a) à des substances polyosidiques ou portant des groupements osidiques (8), pour former le complexe (10a ou 10b), la liaison étant réalisée par l'intermédiaire de la lectine comme indiqué par le schéma C représenté sur la figure 1 annexée.

Les complexes résultants peuvent être utilisés dans tous les procédés où une phase insoluble est nécessaire, comme la chromatographie d'affinité, ou en vue de l'extraction de la caractérisation et/ou du dosage d'un antigène ou d'un anticorps.

L'invention va être maintenant décrite par les exemples non limitatifs ci-après dans lesquels la lectine mise en oeuvre est la Concanavaline A (Con. A).

Exemple 1

Préparation du produit de couplage lectine-anticorps à l'aide de glutaraldéhyde

On a utilisé des anticorps de mouton anti-Ig de lapin purs isolés par chromatographie d'affinité. Les anticorps et la Con. A ont été dialysés contre du tampon phosphate 0,1 M pH 6,8 pendant 1 nuit à 4°C. Les anticorps (2 mg) et la Con. A (4 mg), dialysés, ont été mélangés dans une solution contenant du méthyl-α-D-mannoside (0,1 M). Le volume total était égal à 1 ml. 25 μl d'une solution aqueuse de glutaraldéhyde ont été ajoutés au mélange, qui a ensuite été incubé pendant 3 heures à température ambiante. Puis, on a ajouté 50 μl de glycocolle 2M; le mélange a été laissé 2 heures à la température du laboratoire avant d'être dialysé pendant 24 heures contre une solution de NaCl 0,3 M contenant du CaCl$_2$ (1 mM) et du MnCl$_2$ (1 mM). Après la dialyse et la centrifugation, on a ajouté 1 ml de glycérol, puis la préparation a été conservée dans un congélateur à −20°C.

Exemple 2

Préparation du produit de couplage lectine-anticorps à l'aide de p-benzoquinone

A—par activation des anticorps:

Les anticorps et la Con. A ont été dialysés contre une solution de NaCl 0,15 M.

A la solution d'anticorps (4 mg dans 0,7 ml) on a ajouté successivement 70 μl de tampon phosphate pH

6

**0 041 426**

6 1 M et 100 µl de p-benzoquinone (en solution alcoolique à 40 mg/ml). Après 1 heure d'incubation à l'obscurité et à température ambiante, la préparation a été filtrée sur une colonne de Sephadex G25 (0,9×20 cm). Le premier pic obtenu (1,6 ml) contenait les anticorps activés. Ceux-ci ont été alors mélangés avec 6 mg de Con. A (360 µl). 200 µl de tampon dicarbonate-carbonate 1 M pH 9 ont été ajoutés au mélange. Celui-ci a été maintenu pendant 48 heures à 4°C. La réaction a été arrêtée par l'addition de 100 µl de glycocolle 2M. Après deux heures, le mélange a été dyalisé contre une solution de NaCl 0,30M contenant du $CaCl_2$ (1 mM) et du $MnCl_2$ (1 mM) puis centrifugé. Ensuite, on a ajouté un volume égal de glycérol. La préparation a été conservée à −20°C.

B—par activation de la lectine Con. A

La Con. A préalablement dialysée contre une solution de NaCl 0,15 M a été activée de la façon suivante: à la solution de Con. A (5,4 mg dans 0,6 ml) contenant un tampon phosphate 0,1 M pH 6 et du méthyl-α-D-mannoside 0,1 M, on a ajouté 100 µl de p-benzoquinone (40 mg par ml d'éthanol). Après une heure, la préparation a été filtrée sur Séphadex G25. Le premier pic a été recueilli (1,7 ml) et mélangé avec 0,6 ml d'anticorps (3,5 mg) et 230 µl de tampon bicarbonate-carbonate de 1 M pH 9. Après 48 heures à 4°C, le mélange a été dialysé, centrifugé, puis mélangé avec un volume égal de glycérol. La préparation a été conservée à −20°C.

Exemple 3

Préparation d'un produit de couplage lectine-antigène

On a opéré selon le même mode opératoire que celui décrit à l'exemple 1 en utilisant des immunoglobulines de mouton à la place des anticorps de mouton anti-Ig de lapin.

Dans cet exemple, on a alors utilisé les ingrédients ci-après dans les proportions suivantes:

| Con. A 28,8 mg/ml | Ig de mouton 4,4 mg/ml | Tampon phosphate pH 6,8 1M | Méthyl-α-D-mannoside 1M | Glutaraldéhyde 1% |
|---|---|---|---|---|
| 174 µl=5 mg | 568 µl=2,5 mg | 90 µl | 90 µl | 20 µl |

La solution obtenue a été conservée à −20°C.

Exemple 4

Dosage d'anticorps et d'antigènes

A—dosage d'anticorps

Comme modèle expérimental, on a choisi le système suivant: dosage d'anticorps présents dans des sérums de lapins immunisés avec l'albumine sérique bovine.

Les godets d'une plaque de polystyrène ont été recouverts d'albumine sérique bovine. Après l'adsorption, les godets ont été lavés avec une solution tampon phosphate contenant du "Tween" 20 (1%), dénommée ci-après: PBS-Tween. L'immunsérum de lapin a été dilué de 1/40.000 à 1/10 240.000 dans du PBS contenant de la gélatine (0,5%) et du Tween 20 (1%). Les godets ont reçu 200 µl de chaque dilution. Un témoin a été effectué en remplaçant l'immunsérum par un sérum de lapin nonimmunisé. Après incubation (2 heures à 37°C) et 5 lavages avec du PBS Tween, tous les godets ont reçu 200 µl d'une solution du produit de couplage anticorps de mouton anti-Ig de lapin Con. A (5 µl/ml) préparé selon l'exemple 1 ci-dessus. Le diluant était constitué de NaCl 0,3 M contenant du $CaCl_2$ 1 mM, $MnCl_2$ 1 mM, du Tween (0,5%) de la gélatine (0,5%) et du méthyl-α-D-mannoside. Après 2 heures à 37°C, les godets ont été vidés et lavés avec du PBS Tween. Puis les godets ont reçu 200 µl d'une solution de péroxydase (10 µg/ml) dans du NaCl 0,15 M contenant du "Tween 20" 0,5%, du $CaCl_2$ 1 mM, et du $MnCl_2$ 1 mM. Après l'incubation de 3 heures à 37°C, les godets ont à nouveau été lavés avec de PBS Tween puis remplis par 200 µl de substrat de la peroxydase ($H_2O_2$+o-phénylène diamine). Après 15 minutes, la réaction enzymatique a été arrêtée par l'addition de 50 µl HCl 3N. La densité optique a été mesurée à 492 nm.

Résultats:

**0 041 426**

| Dilution | D0 moyenne |
|---|---|
| 1/40.000 | 2.325 |
| 1/80.000 | 1.477 |
| 1/160.000 | 1.019 |
| 1/320.000 | 662 |
| 1/640.000 | 341 |
| 1/1.280.000 | 181 |
| 1/2.560.000 | 120 |
| 1/5.120.000 | 72 |
| 1/10.240.000 | 23 |
| Témoin: sérum de lapin normal 1/40.000 | 160 |

B—Dosage d'antigènes

En utilisant un mode opératoire similaire à celui décrit ci-dessus, on a dosé de l'IgE humaine. Ce mode opératoire comprend les étapes ci-après:

— Adsorption des anticorps anti-IgE sur une plaque de polystyrène,
— Incubation (2 heures) du sérum du malade à titrer, diluant: PBS contenant du Tween 20 et de l'albumine bovine 1%.
— Incubation (2 heures) avec les anticorps anti-IgE couplés avec la Con. A (5 µl/ml); diluant: PBS contenant 0,1 M de méthyl-α-D-mannoside.
— Incubation (3 heures) avec la peroxydase (10 µl/ml) diluée dans du NaCl 0,15 M contenant 1 mM $CaCl_2$ et 1 mM $MnCl_2$.
— Réaction enzymatique, 30 minutes.
— Mesure de la densité optique à 492 nm.

En suivant le schéma réactionnel, on a pu doser les IgE humaines entre 1 et 500 U.l/ml.

Exemple 5

Titrage d'antigènes par érythroadsorption

Le déroulement du dosage est le même que celui décrit en détails pour le dosage immunoenzymatique dans l'exemple 4 excepté que, lors de la troisième incubation (incubation avec l'enzyme) on remplace l'enzyme par une suspension de globules rouges de mouton à 0,1% non sensibilisés. Après deux heures d'incubation, on note la dernière dilution de l'immun-sérum ou de l'antigène donnant une érythroadsorption encore visible. La spécificité de ce dosage est attestée par la non érythroadsorption lorsqu'on remplace l'immunsérum par un sérum normal, et par l'inhibition de l'adsorption en présence de méthyl-α-D-mannoside. On a donné ci-après les résultats obtenus lors du dosage de l'immun-sérum de lapin anti-albumine bovine.

8

# 0 041 426

## TABLEAU 1

Résultats:

| Immun-sérum de la pin anti-albumine bovine | Erythroadsorption des érythrocytes | Erythroadsorption des érythrocytes en présence de méthyl-α-D-mannoside 0,1 M |
|---|---|---|
| 1/160.000 | +++ | — |
| 1/320.000 | +++ | — |
| 1/640.000 | +++ | — |
| 1/1.280.000 | +++ | — |
| 1/2.560.000 | ++ | — |
| 1/5.120.000 | + | — |
| Sérum normal 1/10.000 | — | — |

Exemple 6

Dosage de l'α-foetoprotéine dans les sérums humains par érythroadsorption

En utilisant un mode opératoire similaire à celui de l'exemple 5, on a dosé l'α-foetoprotéine dans des sérums humains.

Ce mode opératoire comprend les étapes ci-après:

— Adsorption des anticorps anti-α-foetoprotéine sur une microplaque en polystyrène ayant des puits à section en forme de U ou de V.
— Incubation (2 heures) du sérum humain à titrer; diluant: PBS+"Tween" (1%)+albumine bobine (1%),
— Incubation (2 heures) avec des anticorps anti-α-foetoprotéine couplés avec la Con A. (diluant: PBS contenant 0,1 M de méthyl-α-D-mannoside) et "Tween" (1%).
— Incubation (2 heures) avec des globules rouges de mouton,
— détermination de l'érythroadsorption (visuelle).

Ces essais ont été réalisés d'une part avec des sérums humains normaux (No. 1, 2 et 3) ayant au préalable subi une absorption par des globules rouges de mouton afin d'éliminer les éventuels anticorps anti-globules rouges présents dans ces sérums, et d'autre part avec des échantillons de ces mêmes sérums n'ayant pas subi une telle absorption.

Cette absorption a été réalisée selon le mode opératoire défini précédemment dans le cadre d'antigènes non thermolabiles.

A titre de référence, on a utilisé un sérum standard (le sérum de cordon) ayant une concentration en α-foetoprotéine de 66 µg/ml.

Les résultats obtenus sont consignés dans le tableau ci-après:

TABLEAU 2

| Dilution du sérum standard (sérum de cordon) | Erythroadsorption sans absorption préalable du sérum par des globules rouges de mouton | Erythroadsorption avec absorption préalable de sérum par des globules rouges de mouton |
|---|---|---|
| 1/500 | ++++ | ++++ |
| 1/1500 | ++++ | ++++ |
| 1/4500 | +++ | +++ |
| 1/13.500 | — | — |
| 1/40.500 | — | — |

| Dilution du sérum humain à tester | Sérum No. 1 | Sérum No. 2 | Sérum No. 3 | Sérum No. 1 | Sérum No. 2 | Sérum No. 3 |
|---|---|---|---|---|---|---|
| 1/3 | ++++ | ++++ | ++++ | ± | — | — |
| 1/9 | +++ | ++++ | ++++ | — | — | — |
| 1/27 | ++ | ++ | ++++ | — | — | — |
| 1/81 | ± | — | ++++ | — | — | — |
| 1/243 | ± | — | +++ | — | — | — |

Les sérums No. 1, 2 et 3 sont des sérums normaux, c'est-à-dire des sérums qui ne contiennent pas ou très peu d'α-foetoprotéine. Les résultats du tableau 2 ci-dessus montrent que le procédé de l'invention peut donner des réactions faussement positives si on ne prend pas soin d'absorber au préalable les sérums avec des globules rouges de mouton, si ce sont ces globules rouges qui sont utilisés comme marqueurs dans le procédé de l'invention.

Exemple 7

Détection immunocytochimique d'immunoglobulines des immunocytes à l'aide du produit de couplage lectine-anticorps

Des suspensions de lymphocytes provenant de divers organes lymphoides ont été cytocentrifugées sur lame ou des frottis cellulaires ont été effectués à partir de ces suspensions. Les préparations cellulaires ont ensuite été fixées et ensuite incubées dans du PBS contenant 10 µg/ml d'anticorps de mouton anti-immunoglobulines de souris couplés avec la Concanavaline A et du méthyl-α-D-mannoside 0,1 M. Après une heure d'incubation, les préparations cellulaires ont été lavées au PBS puis incubées avec du PBS contenant de la peroxydase de raifort ou de la glucose oxydase à une concentration de 100 µg/ml. Après 2 heures d'incubation à la température de laboratoire, les lames ont été lavées et l'enzyme associée aux cellules a été révélée par une coloration histochimique spécifique de l'enzyme utilisée comme marqueur.

Exemple 8

Cet exemple illustre l'utilisation d'une substance marquée par un fluorochrome, à savoir la glucose-oxydase marquée par l'isothiocyanate de fluorescéine.

(a) En vue de marquage de la glucose-oxydase par la fluorescéine, on procède comme suit:

On dissout 3 mg de glucose-oxydase dans 140 µl de NaCl 0,15 M. On agite et on ajoute ensuite 48 µl d'isothiocyanate de fluorescéine (solution à 10 mg/ml dans du bicarbonate 1 M pH 9,5). On laisse 4 heures à la température du laboratoire. On passe le produit obtenu sur une colonne remplie d'Ultrogel Ac-A-202 (marque déposée par Industrie Biologique Française—Pharmaindustrie) préalablement lavée avec du NaCl 0,15 M. On élue avec la même solution de NaCl et on recueille les fractions contenant la glucose-oxydase marquée par la fluorescéine.

(b) le déroulement du dosage d'anticorps et d'antigène est identique à celui précédemment décrit. La révélation de la présence du conjugé anticorps-Con. A se fait par incubation avec la glucose-oxydase rendue fluorescente. Le mesure de la fluorescence est effectuée soit dans le surnageant après l'incubation soit dans l'éluat après traitement de la phase solide par un réactif dissociant, tel que l'urée, le dodécyl-sulfate de sodium, des tampons acides ou alcalins, des solutions concentrées d'acide fort (HCl 6N)

# 0 041 426

ou de base forte (NaOH 6N), et d'autres produits analogues, ou par le sucre spécifique de la lectine (exemple méthyl-α-D-mannoside 1M).

Des résultats d'essais concrets réalisés avec la glucose-oxydase marquée par la fluorescéine sont présentés dans le tableau 3 suivant.

### TABLEAU 3

| Dilution de la glucose oxydase marquée par la fluorescéine | Fluorescence (unité de fluorescence) | | |
|---|---|---|---|
| | Dans la solution de départ | Dans le surnageant après incubation | Dans le godet ≠ |
| 1/100 | 155 UF | 150 | 2,9 |
| 1/500 | 35 | 27 | 2,6 |
| 1/1.000 | 19 | 12 | 2,4 |

≠ mesurée après dissociation par du méthyl-α-D-mannoside (1M) de la liaison entre la Con-A et la glucose oxydase marquée.

## Exemple 9

Cet exemple illustre l'utilisation d'une substance marquée par un isotope radioactif, à savoir la glucose oxydase marquée par l'$^{125}$I.

La technique utilisée à l'exemple 7 pour la fluorescence est applicable dans ce système, mais la mesure de la radioactivité dans les godets, sur la phase solide, remplace la mesure de la fluorescence.

Des résultats d'essais concrets réalisés avec la glucose oxydase marquée à l'$^{125}$I sont présentés dans le tableau 4 qui suit:

### TABLEAU 4
Radioactivé mesurée pour chaque godet
après élution par NaOH 6N

| Dilution de la glucose oxydase marquée par l'$^{125}$I | Ac Con. A≠ 1/100 | Ac Con. A≠ 1/1000 | Ac Con. A≠ 0 |
|---|---|---|---|
| 1/10 | 45.000 cpm | 7.500 cpm | 350 |
| 1/30 | 30.000 cpm | 5.700 cpm | 210 |
| 1/90 | 6.500 cpm | 3.000 cpm | — |
| 1/270 | 2.400 cpm | 550 cpm | 90 |
| 1/810 | 1.000 cpm | 200 cpm | — |

≠ Ac Con. A=Anticorps couplé par le glutaraldéhyde avec la Concanavaline A.

## Exemple 10

Cet exemple illustre la technique de lecture par hémolyse.

Après l'érythroadsorption spécifique, les globules rouges n'ayant pas réagi avec le réactif (Anticorps-lectine) décantent et tombent dans le fond des godets. Les globules rouges décantés sont alors aspirés doucement avec une pipette.

Les globules rouges liés au réactif (Anticorps-lectine) restent dans les godets. On ajoute de l'eau distillée ou autre liquide permettant de lyser les globules rouges. La lyse est immédiate. La lecture se fait par absorption à 403 nm de l'hémoglobine libérée.

Des résultats d'essais concrets sont présentés dans le tableau 5 qui suit:

11

# 0 041 426

### TABLEAU 5

| Dilution de conjugués anticorps≠-lectine≠≠ | $^{DO}$403 nm |
|---|---|
| 1/500≠≠≠ | 488 |
| 1/1000 | 365 |
| 1/2000 | 336 |
| 1/4000 | 272 |
| 1/8000 | 217 |
| 1/16.000 | 133 |
| 1/32.000 | 88 |
| Témoin sans conjugué | 83 |

≠ l'anticorps était l'antiimmunoglobuline de lapin

≠≠ la lectine était la Concanavaline A.

≠≠≠ pour la dilution au 1/500, la concentration était de 2 µg/ml d'anticorps et 4 µg/ml de Concanavaline A.

Exemple 11

Titrage d'un immunsérum de lapin anti-albumine sérique bovine par érythroadsorption en utilisant un conjugué anticorps—Con A

On a réalisé ce titrage en utilisant un mode opératoire similaire à ceux décrits dans les exemples 5 et 6 ci-dessus.

Ce mode opératoire comprend les étapes suivantes:

— Adsorption de l'albumine sérique bovine sur les microplaques en polystyrène à puits en forme de U ou de V.

— Incubation avec l'immunsérum de lapin.

— Incubation avec le conjugué anticorps-Con. A obtenu selon le mode opératoire décrit à l'exemple 1, l'anticorps étant l'anticorps de mouton anti-Ig de lapin.

— Incubation avec des globules rouges de mouton.

— Détermination de l'érythroadsorption.

La détermination de l'érythroadsorption a été effectuée de trois façons différentes:

1) visuellement ou

2) après hémolyse des globules rouges par mesure de la densité optique à 405 nm.

3) après hémolyse, puis réaction enzymatique en utilisant comme substrat l'ortho-phénylène-diamine, par mesure de la densité optique à 492 nm.

Les résultats obtenus sont consignés dans le tableau 6.

La spécificité d'un tel dosage mettant en oeuvre le conjugué selon l'invention a été démontrée par l'inhibition totale en pré-incubant l'immunsérum anti-albumine sérique bovine avec un excès d'albumine sérique bovine.

Exemple 12

Dosage par érythroadsorption en utilisant un conjugué anticorps—WGA

On a utilisé un conjugué anticorps-WGA obtenu selon le mode opératoire décrit à l'exemple 1 et on a effectué le dosage par érythroadsorption des anticorps ou antigènes ci-après en opérant sensiblement selon les modes opératoires décrits dans les exemples 5 et 6 ci-dessus.

— IgE humaine totale,

— IgE humaine spécifique anti-pollen de graminées

— anticorps anti-CMV (cytomégalovirus)

— anticorps antihydratidose

— anticorps anticandida albicans

— anticorps antiaspergillus fumigatus et on a comparé les résultats obtenus à ceux obtenus par des techniques immunologiques classiques, telles que le dosage quantitatif radio-immunologique pour l'IgE humaine totale et l'IgE humaine spécifique anti-pollen de graminées, l'hémagglutination passive pour

12

# 0 041 426

les anticorps antihydatidose et les anticorps anti-CMV et l'électrosynérèse pour les anticorps anticandida albicans et les anticorps antiaspergillus fumigatus.

Dans chaque cas on a trouvé une bonne corrélation entre le dosage par érythroadsorption selon l'invention et la technique classique, avec un coefficient de corrélation statistique significatif.

## TABLEAU 6

| Dilution de l'immunsérum de lapin anti-albumine bovine | Lecture visuelle | Détermination de l'érythroadsorption | |
|---|---|---|---|
| | | Hémolyse puis lecture de la D.O. à 405 nm | Hémolyse puis réaction enzymatique et D.O. à 492 nm |
| 1/10.000 | ++++ | 0,177 | 0,986 |
| 1/20.000 | ++++ | 0,180 | 0,981 |
| 1/40.000 | ++++ | 0,167 | 0,847 |
| 1/80.000 | ++++ | 0,153 | 0,834 |
| 1/160.000 | +++ | 0,141 | 0,762 |
| 1/320.000 | ++ | 0,098 | 0,555 |
| 1/640.000 | + | 0,078 | 0,453 |
| 1/1.280.000 | ± | 0,042 | 0,278 |
| 1/2.560.000 | − | 0,004 | 0,086 |
| 1/5.120.000 | − | 0,000 | 0,044 |
| sérum de lapin normal 1/10.000 | − | 0,000 | 0,007 |

## Revendications

1. Produit de couplage par liaisons covalentes, soluble en milieu aqueux, entre une lectine et un ligand spécifique choisi parmi les antigènes, les anticorps, les haptènes, les hormones ou leurs récepteurs, les enzymes ou leurs inhibiteurs, les lectines.

2. Produit de couplage selon la revendication 1, caractérisé en ce qu'il est soluble en milieu aqueux en présence d'un sucre de faible poids moléculaire spécifique de la lectine.

3. Produit de couplage selon l'une des revendications 1 ou 2, caractérisé en ce que la lectine est la Concanavaline A, la Ricine, la lectine extraite de *Triticum vulgare* c'est-à-dire l'agglutinine de germe de blé on la lectine extraite de *Lens culinaris*.

4. Produit de couplage selon l'une quelconque des revendications 1 à 3, caractérisé en ce qu'il est obtenu par couplage à l'aide de glutaraldéhyde.

5. Produit de couplage selon l'une quelconque des revendications 1 à 3, caractérisé en ce qu'il est obtenu par couplage à l'aide de/la benzoquinone en deux étapes, la première consistant à activer la lectine ou le ligand avec la benzoquinone et la seconde à faire incuber le produit résultant avec le second produit à coupler.

6. Procédé de dosage immunologique d'une substance biologique, caractérisé en ce qu'il consiste:

1) à immobiliser une substance ayant une affinité de fixation pour la substance biologique à doser;

2) à faire incuber cette substance immobilisée avec le milieu contenant la substance biologique à doser;

3) à faire incuber, après lavage, le milieu réactionnel résultant avec un produit de couplage selon l'une quelconque des revendications 1 à 5, en solution dans un milieu aqueux contenant un excès d'un sucre spécifique de la lectine, le ligand spécifique étant capable de réagir de façon spécifique avec ladite substance ayant une affinité pour la substance biologique à doser ou avec ladite substance biologique elle-même,

4) à laver le milieu réactionnel résultant et à le faire incuber avec un marqueur portant des fractions glycosidiques pouvant réagir avec la lectine; et

5) à révéler le marqueur par un moyen approprié.

13

7. Procédé selon la revendication 6, caractérisé en ce que la marqueur est une enzyme, une substance portant un isotope radioactif ou un fluorochrome, ou une substance particulaire apte à réagir avec la lectine.

8. Procédé selon la revendication 6, caractérisé en ce que le marqueur est constitué par des globules rouges.

9. Application du procédé de dosage selon l'une des revendications 6 à 8 à la localisation, la mise en évidence et le dosage quantitatif d'un antigène ou d'un anticorps par fluorimétrie, radioimmunologie, immunoenzymologie, érythroadsorption.

10. Procédé selon l'une des revendications 6 à 8, caractérisé en ce que pour révéler, selon l'étape 5 de la revendication 6, les globules rouges utilisés à titre de marqueur on élimine les globules rouges n'ayant pas réagi, on réalise une lyse des globules rouges fixés sur le produit de couplage lectine-ligand et on dose les substances libérées par les globules rouges.

11. Procédé selon la revendication 10, caractérisé en ce que la lyse est effectuée avec de l'eau distillée.

12. Procédé selon l'une quelconque des revendications 6, 8, 10 ou 11, caractérisé en ce que le milieu liquide contenant la substance biologique à doser est un sérum et en ce que le sérum est préalablement à l'étape 2) de la revendication 6, absorbé par des globules rouges.

13. Procédé selon la revendication 12, caractérisé en ce que le sérum est absorbé par des globules rouges selon le mode opératoire qui comprend les étapes consistant:

1) à décomplémenter le sérum par chauffage à 56°C environ pendant 30 minutes environ;

2) à ajouter un excès de globules rouges de mouton audit sérum, par exemple pour obtenir une concentration en globules rouges de 10%;

3) à laisser le sérum ainsi traité à la température ambiante pendant environ 1 heure sous agitation douce; et

4) à centrifuger pour récupérer le sérum purifié et éliminer le culot de globules rouges.

14. Procédé selon la revendication 13, caractérisé en ce que le sérum est absorbé par des globules rouges traités par un agent tannant, tel que le glutaraldéhyde.

**Patentansprüche**

1. In Wasser lösliches Kupplungsprodukt durch kovalente Bindungen zwischen einem Lectin und einem spezifischen Liganden, ausgewählt unter den Antigenen, den Antikörpern, den Haptenen, den Hormonen oder ihren Rezeptoren, den Enzymen oder ihren Inhibitoren und den Lectinen.

2. Kupplungsprodukt nach Anspruch 1, dadurch gekennzeichnet, daß es in Gegenwart von Zucker mit einem niedrigen, für Lectin spezifischen Molekulargewicht, in Wasser löslich ist.

3. Kupplungsprodukt nach einem der Ansprüche 1 oder 2, dadurch gekennzeichnet, daß das Lectin Concanavalin A, Ricin, aus Triticum vulgare extrahiertes Lectin, d.h. Agglutinin aus dem Fruchtknoten von Getreide, oder extrahiertes Lectin aus Lens culinaris ist.

4. Kupplungsprodukt nach einem der Ansprüche 1 bis 3, dadurch gekennzeichnet, daß es durch Kupplung mit Hilfe von Glutaraldehyd erhalten worden ist.

5. Kupplungsprodukt nach einem der Ansprüche 1 bis 3, dadurch gekennzeichnet, daß es durch Kupplung mit Hilfe von Benzochinon in zwei Stufen erhalten wird, wobei die erste in der Aktivierung des Lectin oder des Liganden mit Benzochinon und die zweite in der Inkubierung des resultierenden Produktes mit dem zweiten zu kuppelnden Produkt besteht.

6. Verfahren zur ummunologischen Bestimmung einer biologischen Substanz, dadurch gekennzeichnet, daß es besteht aus:

(1) dem Immobilisieren einer Substanz mit einer Fixierungsaffinität für die zu bestimmende biologische Substanz,

(2) dem Inkubieren dieser immobilisierten Substanz mit dem Medium, das die zu bestimmende biologische Substanz enthält,

(3) dem Inkubieren nach dem Waschen des entstehenden Reaktionsmediums mit einem Kupplungsprodukt gemäß irgendeinem der Ansprüche 1 bis 5 in einer Lösung in einem wässrigen Medium, das einen Überschuß eines für das Lectin spezifischen Zuckers enthält, wobei der spezifische Ligand fähig ist, in spezifischer Weise mit der besagten Substanz, die eine Affinität für die zu bestimmende biologische Substanz besitzt, oder mit der besagten biologischen Substanz selbst zu reagieren,

(4) dem Waschen des entstehenden Reaktionsmediums und dem Inkubieren mit einem Markierungsmittel, das glykosidische Gruppen trägt, die mit dem Lectin reagieren können und

(5) dem Sichtbarmachen des Markierungsmittels durch ein entsprechendes Mittel.

7. Verfahren nach Anspruch 6, dadurch gekennzeichnet, daß das Markierungsmittel ein Enzym, eine Substanz, die ein radioaktives Isotop oder eine fluoreszierende Gruppe trägt, oder eine besondere Substanz ist, die befähigt ist, mit dem Lectin zu reagieren.

8. Verfahren nach Anspruch 6, dadurch gekennzeichnet, daß das Markierungsmittel durch rote Blutkörperchen gebildet wird.

9. Anwendung des Bestimmungsverfahrens nach einem der Ansprüche 6 bis 8 zur Lokalisierung, zum Sichtbarmachen und zur quantitativen Bestimmung von Antigenen oder Antikörpern durch Fluorometrie, Radioimmunologie, Immunoenzymologie und Erythroadsorption.

10. Verfahren nach einem der Ansprüche 6 bis 8, dadurch gekennzeichnet, daß man für das Sichtbarmachen der zum Titrieren des Markierungsmittels benutzten roten Blutkörperchen die roten Blutkörperchen eliminiert, die nicht reagiert haben, man die mit dem Kupplungsprodukt Lectin-Ligand fixierten roten Blutkörperchen löst, und die von roten Blutkörperchen freien Substanzen bestimmt.

11. Verfahren nach Anspruch 10, dadurch gekennzeichnet, daß das Lösen mit destilliertem Wasser durchgeführt wird.

12. Verfahren nach irgendeinem der Ansprüche 6, 8, 10 oder 11, dadurch gekennzeichnet, daß das flüssige Medium, das die zu bestimmende biologische Substanz enthält, ein Serum ist, und daß das Serum vor Stufe (2) des Anspruchs 6 durch rote Blutkörperchen absorbiert wird.

13. Verfahren nach Anspruch 12, dadurch gekennzeichnet, daß das Serum durch die roten Blutkörperchen nach der Arbeitsmethode absorbiert wird, die aus den Stufen besteht:

(1) dem Auftrennen des Serums durch Hitze bei ungefähr 56°C während ungefähr 30 Minuten,

(2) dem Hinzufügen eines Überschusses roter Blutkörperchen vom Schaf zum Serum, z.B. um eine Konzentration an roten Blutkörperchen von 10% zu erhalten,

(3) dem Belassen des so behandelten Serums bei Umgebungstemperatur für 1 h unter leichtem Rühren und

(4) dem Zentrifugieren, um das gereinigte Serum wiederzugewinnen und den Rückstand der roten Blutkörperchen abzutrennen.

14. Verfahren nach Anspruch 13, dadurch gekennzeichnet, daß das Serum durch rote Blutkörperchen absorbiert wird, die durch ein Gerbmittel, wie Glutaraldehyd behandelt worden sind.

## Claims

1. A water-soluble conjugate, obtained by covalent coupling of a lectin with a specific ligand selected from antigens, antibodies, haptens, hormones and their receptors, enzymes and their inhibitors, lectins.

2. A conjugate as claimed in claim 1, characterized in that it is water-soluble in a medium containing a sugar specific to the lectin, having a low molecular weight.

3. A conjugate as claimed in claim 1 or 2, characterized in that the lectin is selected from the group consisting of Concanavalin A, Ricin, *Triticum vulgare* lectin, i.e. wheat eye agglutinin, or *Lens culinaris* lectin.

4. A conjugate as claimed in any claim 1 to 3, characterized in that it is obtained by coupling with glutaraldehyde.

5. A conjugate as claimed in any claim 1 to 3, characterized in that it is obtained by coupling with benzoquinone in a two step reaction, the first step comprising activating ligand or lectin with benzoquinone and second step comprising incubating the reaction product with the second product to be coupled.

6. A method for the immunological assay of a biological substance, characterized in that it consists of:

1) immobilizing a substance having a binding affinity for said biological substance to be determined;

2) incubating said substance with a medium containing said biological substance to be determined;

3) incubating, after washing, the resulting reaction medium with a conjugate according to any claim 1 to 5, in solution in an aqueous medium containing an excess of a sugar specific to the lectin, said ligand being capable of reacting specifically with said substance having an affinity to the biological substance to be assayed or with said biological substance itself;

4) washing the resulting reaction medium and incubating it with a labeller bearing glycosidic fractions capable of reacting with said lectin; and

5) assaying said labeller by suitable means.

7. Process as claimed in claim 6, characterized in that the labeller is an enzyme, a radioactive compound, a fluorochromic compound, or a particulate substance able to combine with the lectin.

8. A method as claimed in claim 6, characterized in that the labeller consists in red blood cells.

9. Use of the method according to any claim 6 to 8 for locating, detecting, or quantitatively determining antigen, or antibody with fluorimetric method, radioimmunoassay, immunoenzymologic assay or adsorption on red blood cells.

10. A method as claimed in any claim 6 to 8 characterized in that the assay of red blood cells used as the labeller, cited in step 5 of claim 6, consists of: eliminating the unreacted red blood cells, lysing the red blood cells bound to the lectin-ligand conjugate and determining products released from red blood cells.

11. A method as claimed in claim 10, characterized in that lysis is produced by distilled water.

12. A method as claimed in any claim 6, 8, 10 or 11 characterized in that the liquid medium comprising the biological substance to be determined is a serum and that said serum before step 2 of claim 6, is absorbed with red blood cells.

13. A method as claimed in claim 12, characterized in that the serum is absorbed with red blood cells according to the following process which consists of:

1) decomplementing the serum by heating at about 56°C for about 30 minutes;

2) adding an excess of sheep red blood cells to said serum, in order to obtain a concentration of red blood cells of 10%;

3) leaving said serum at ambient temperature for about one hour under smooth stirring; and

4) centrifuging in order to recover the purified serum and, remove the bottom of red blood cells.

14. Process as claimed in claim 13, characterized in that serum is absorbed with red blood cells, treated with a tanning agent, such as glutaraldehyde.

SCHEMA A

1   2   3   4

5   6   7

▸ antigène

⊢⟶ antigène immobilisé

⟩⟶⟨ conjugué Anticorps-Lectine

o sucre de faible poids moléculaire réagissant avec la lectine.

◁▭ substance marqueur pouvant réagir avec la lectine.

SCHEMA B

1   2   3   4

5   6   7

▸ antigène

⊢⟶ antigène immobilisé

⟩⟶⟨ conjugué Anticorps-Lectine

◁▭ substance marqueur pouvant réagir avec la lectine.

SCHEMA C

8 + 9a ⟶ 10a

8 + 9b ⟶ 10b

FIG.1

⟩ substance polyosidique

⟩⟶⟨ anticorps marqué par une lectine.

⟩⟶ antigène marqué par une lectine.

0 041 426

1

FIG.2